# EUROPEAN PATENT SPECIFICATION

(11) **EP 3 402 570 B1**
(45) Date of publication and mention of the grant of the patent: **27.05.2020**
(21) Application number: 15823403.9
(22) Date of filing: 30.11.2015
(51) Int. Cl.: A61N 5/06

(54) **COMPACT UVB LIGHT THERAPY DEVICE FOR TREATING DERMAL CONDITIONS**
KOMPAKTE UVB-LICHTTHERAPIE-VORRICHTUNG ZUR BEHANDLUNG VON HAUTERKRANKUNGEN
DISPOSITIF DE THÉRAPIE À LUMIÈRE UVB COMPACT POUR TRAITER DES TROUBLES DERMIQUES

(43) Date of publication of application: 21.11.2018
(73) Proprietor: Comin SPOLKA Z OGRANICZONA ODPOWIEDZIALNOSCIA, 01-737 Warszawa (PL)
(72) Inventor: SZOSTKIEWICZ, Lukasz, 97-100 Torun (PL); NAPIERALA, Marek, 01-476 Warszawa (PL); NASILOWSKI, Tomasz, 00-172 Warszawa (PL); STANCZYK, Tomasz, 05-500 Piaseczno (PL); KUKLINSKA, Malgorzata, 20-455 Lublin (PL); MAKOWSKA, Anna, 01-884 Warszawa (PL); PYTEL, Anna, 02-790 Warszawa (PL); PAWLIK, Katarzyna, 03-126 Warszawa (PL); HOLDYNSKI, Zbigniew, 01-129 Warszawa (PL); LIPINSKI, Stanislaw, 13-240 Ilowo (PL); FILIPOWICZ, Marta, 05-400 Otwock (PL); WAJNCHOLD, Barbara, 33-176 Kraków (PL)
(74) Representative: Bury & Bury
(86) International application number: PCT/PL2015/050064
(87) International publication number: WO 2017/095246

(56) References cited:
- US-A1- 2006 167 531
- US-A1- 2006 206 173

## Description

The subject of the invention is a compact UVB light therapy device, particularly intended for the treatment of vitiligo, atopic dermatitis and psoriasis.

Light therapy, also referred to as phototherapy, is a rapidly developing field of medicine and pharmaceutics. Contemporary phototherapy applies a wide spectrum of visible light frequencies, plus the ultraviolet and infrared ranges. The phototherapeutic psoriasis, atopic dermatitis and vitiligo treatment method applies UV radiation sources. According to various sources, the global incidence of vitiligo is 1-2% of the entire population, approximately 30% of the population suffers from atopic dermatitis, and psoriasis is a menace to 2-4% of the population. Currently, science distinguishes three types of UV radiation suitable for treating the aforementioned diseases: PUVA therapy, Broadband UVB and Narrow band UVB.

The PUVA method assumes the application of type A ultraviolet rays in a band of 340- 400 nm. UVA radiation is often accompanied by the administration of photosensitizing drugs, out of which the most popular one is psoralen (a natural compound, found in specific plants). In another possible treatment method, drugs are not administered, and the volume of phototherapy is increased. Application of this method must be performed in dedicated clinics three times a week. Human skin is very resistant to UVA radiation, since it reaches us from the sun and, in order for the treatment to bring the desired results, we need drug-assisted therapy or increased radiation powers. Due to the fact that UVA radiation passes through glass and can penetrate the body, we need special ocular protection and suitable covers of body parts which need to be excluded from radiation.

The broadband method applies 290-315 nm radiation. UVB radiation is generally considered to be safer, as it does not easily pass through human skin and does not constitute as much of a threat to UVA. In addition, due to increased energy capacity of the radiation, it is possible to apply shorter exposure times, which reduces the patient's exposure to the negative effects of UV radiation. This method required supplementary drug therapy. Depending on the severity of the disease, the patient should undergo radiation 2-5 times a week. There are some available ready-to-use solutions on the market, which allow for conducting the therapy at home. One of the disadvantages of such therapy is that it can cause erythema, as it produces wavelengths which are dangerous to human health, i.e. above 290 nm.

Narrow band UVB therapy is a latest development, producing very promising results in cosmetic and medical applications. 311-312 nm UVB rays are used in the procedure, as they are the most effective for medical applications. One of the advantages of narrowband sources is the absence of radiation below 290 nm. This method required however drug administration. It is more effective and more efficient in producing the desired effects, compared to the broadband UVB method. This method can be applied locally, for certain body parts, and for the entire body.

Patent ref. UA99068 describes a light therapy method, where particular body parts are exposed to 310 nm UV rays. For the duration of therapy, the dosage of radiation is increased by 20%. After that, the patient receives 1 ml of solution comprising stem cells and continues the light treatment, beginning with light of 80mJ/cm² in total energy. Patent solution ref. RU2472541, registered in Russia in 2011, displays a method of treating acne, consisting in the illumination of skin with UV A and B light. In addition to the light therapy, the patient receives 10 mg of liptonorm for 21-28 days.

A device according to patent ref. EP1420741 contains a base unit and at least one head unit attached to it. Particular elements of the device are adjusted to different UV light therapies, LLLT, ultrasound therapy, electrostimulation therapy, cryotherapy, and heat therapy using various diodes.

A multifunctional light therapy device is also known from patent description ref. CN 103801007, which introduces a device equipped with at least one insolation head unit, which is furnished with LED diodes emitting infrared or UV light.

Patent description ref. US6471716 presents a device containing a matrix comprising numerous LED diodes emitting light of a wavelength corresponding to the near infrared range.

In reference document US2006167531 was disclosed an optical therapy device for providing therapeutic light to a person's nasal cavity includes a body and at least one UV light source in or on the body. A distal end of the body is configured to be inserted into the person's nasal cavity. The body can be configured to be hand-held. The optical therapy device is configured such that the UV light source emits a dose of UV light toward tissue in the patient's nasal cavity.

Patent description ref. US2006206173 presents a method and apparatus for treating a target body surface using a radiation applicator. The radiation applicator includes a radiation source in combination with a delivery applicator. The applicator has a low profile enable the patient to apply the applicator to a target area underneath, for example, clothing. The applicator can be configured to use one or more radiation sources to apply one or more types of radiation for one or more periods of time. Additionally, the applicator can be configured with a feedback loop to determine when a therapeutically desirable amount of radiation has been delivered.

A compact UVB light therapy device, for vitiligo, atopic dermatitis and psoriasis treatment according to the invention has a casing containing a power supply with a serially connected power switch having a timer unit, wherein at least one LED light source is connected to power switch with a wire. The at least one LED light source is adapted to emit radiation at a wavelength of 310 nm with spectral full width at half maximum up to 10 nm, and has an optical power in the range of 0.6 mW to 1 mW. The device has a detachable spacer having a height of 1 cm covering the LED light source and fixed to the casing in the vicinity of the LED light source.

Device according to the invention is devoid of any of the limitations related to the other available methods. The solution developed is suitable for use by individual clients, without the need to perform elaborate procedures in cosmetic salons or medical clinics. The device is handy, small and can therefore be used when performing other activities. Furthermore, the device is suitable for use at higher frequencies. Frequent therapy involving lower light concentrations is more effective than a smaller number of high-power insolation sessions. The device according to the invention contains at least 1 LED emitting 310 nm radiation, a spacer and a transparent cover for UVB rays, to maintain the proper distance from the insolated spot and to protect the LED against dust and impurities, plus signaling LEDs, a timer counting the session time, a min. 1A charger, a power switch and two other switches. The signaling diodes include 1 red diode, which lights up when the device is charging, and which goes out when the device is fully charged, 2 blue diodes, which light up together with the UV diodes, 1 green diode, which illuminates in continuous light when the power is on, or blinks every 1 sec. when the batteries are low.

In a beneficial embodiment, at least one additional visible light LED, preferably blue, is installed in the vicinity of the UVB light source, allowing for the observation of the insolation area. In a beneficial embodiment, at least one additional battery charging or device state diode is connected to the power supply and power switch circuit. In a beneficial embodiment, the power switch of the device is doubled in a manner that its circuits provide independent power supply to UVB diodes and visible light diodes allowing for the observation of the insolated area.

Advantageously the LED light source has 0.8 mW in optical power.

Advantageously in the vicinity of the LED light source there is installed an additional visible light LED that preferably emits blue light.

The device according to any preceding claims, characterized in that an additional battery charging or device state diode is connected to the power supply and power switch circuit.

Advantageously the power switch is doubled to provide independent power supply to the LED light source and additional visible light LED.

Device advantageously is powered from an in-built battery or a charger of at least 1A. Preferably the timer controlling unit is adapted to set LED light source illumination period to 300 s or 600 s with the accuracy of 0.5 s.

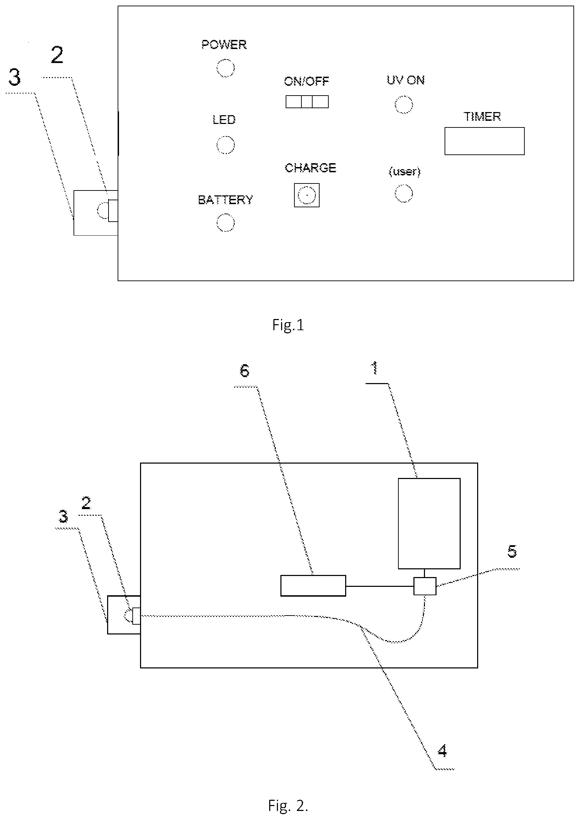

The device according to the invention is described below with reference to the attached drawings, in which fig. 1 illustrates the layout of the device according to the invention, fig. 2 illustrates the cross-section of the device according to the invention.

### Example 1

The compact UVB light therapy device, particularly intended for vitiligo, atopic dermatitis and psoriasis treatment contains an enclosed, combined light source 1, a serially connected power switch 5 with a timer unit 6, and at least one light source - a LED 2 emitting radiation of 310 nm in wavelength and 0.8 mW and higher in optical power, connected to the timer 6, and a detachable spacer 3 covering the light source is fixed in the vicinity of the light source, whereas the spacer is 1 cm high, as measured from the surface of the casing in the vicinity of the light source to the external surface of the spacer. The timer 6 allows for setting the LED illumination period for 600 sec.

An additional visible light LED, preferably blue, is installed in the vicinity of the UVB light source, allowing for the observation of the insolation area. An additional battery charging or device state diode is connected to the power supply and power switch circuit.

The power switch of the device is doubled in a manner that its circuits provide independent power supply to UVB diodes and visible light diodes allowing for the observation of the insolated area.

The device is powered from an in-built battery or a charger of at least 1A.

### Example 2

The compact UVB light therapy device, particularly intended for vitiligo, atopic dermatitis and psoriasis treatment contains an enclosed, combined light source 1, a serially connected power switch 5 with a timer unit 6, and at least one light source - a LED 2 emitting radiation of 310 nm in wavelength and 0.8 mW and higher in optical power, connected to the timer 6, and a detachable spacer 3 covering the light source is fixed in the vicinity of the light source, whereas the spacer is at least 0.5 cm high, as measured from the surface of the casing in the vicinity of the light source to the external surface of the spacer. The timer 6 allows for setting the LED illumination period for 600 sec.

An additional visible light LED, preferably blue, is installed in the vicinity of the UVB light source, allowing for the observation of the insolation area. An additional battery charging or device state diode is connected to the power supply and power switch circuit. The power switch of the device is doubled in a manner that its circuits provide independent power supply to UVB diodes and visible light diodes allowing for the observation of the insolated area.

The device is powered from an in-built battery or a charger of at least 1A.

### Example 3

The compact UVB light therapy device, particularly intended for vitiligo, atopic dermatitis and psoriasis treatment contains an enclosed, combined light source 1, a serially connected power switch 5 with a timer unit 6, and at least one light source - a LED 2 emitting radiation of 310 nm in wavelength and 0.8 mW and higher in optical power, connected to the timer 6, and a detachable spacer 3 covering the light source is fixed in the vicinity of the light source, whereas the spacer is at least 1 cm high, as measured from the surface of the casing in the vicinity of the light source to the external surface of the spacer. The timer 6 allows for setting the LED illumination period for 300 sec.

An additional visible light LED, preferably blue, is installed in the vicinity of the UVB light source, allowing for the observation of the insolation area. An additional battery charging or device state diode is connected to the power supply and power switch circuit.

The power switch of the device is doubled in a manner that its circuits provide independent power supply to UVB diodes and visible light diodes allowing for the observation of the insolated area. The device is powered from an in-built battery or a charger of at least 1A. Example 4

The compact UVB light therapy device, particularly intended for vitiligo, atopic dermatitis and psoriasis treatment contains an enclosed, combined light source 1, a serially connected power switch 5 with a timer unit 6, and at least one light source - a LED 2 emitting radiation of 310 nm in wavelength and 0.8 mW and higher in optical power, connected to the timer 6, and a detachable spacer 3 covering the light source is fixed in the vicinity of the light source, whereas the spacer is at least 0.5 cm high, as measured from the surface of the casing in the vicinity of the light source to the external surface of the spacer. The timer 6 allows for setting the LED illumination period for 600 sec. An additional visible light LED, preferably blue, is installed in the vicinity of the UVB light source, allowing for the observation of the insolation area. An additional battery charging or device state diode is connected to the power supply and power switch circuit.

The power switch of the device is doubled in a manner that its circuits provide independent power supply to UVB diodes and visible light diodes allowing for the observation of the insolated area.

The device is powered from an in-built battery or a charger of at least 1A.

## Claims

1. A compact UVB light therapy device for treating vitiligo, atopic dermatitis and psoriasis, the device having a casing containing a power supply with a serially connected power switch (5) having a timer unit (6), wherein at least one LED light source (2) is connected to the power switch (5) with a wire (4), wherein the at least one LED source (2) is adapted to emit radiation at a wavelength of 310 nm with a spectral full width at half maximum up to 10 nm and has an optical power in the range of 0.6 mW to 1 mW, and wherein the device has a detachable spacer (3) covering the at least one LED light source (2), being fixed to the casing in the vicinity of the at least one LED light source and having a height of 0.5-1 cm as measured from the surface of the casing to the external surface of the spacer.

2. The device according to claim 1, **characterized in that** the LED light source (2) has 0.8 mW in optical power.

3. The device according to claim 1, **characterized in that** in the vicinity of the LED light source (2) there is installed an additional visible light LED.

4. The device according to claim 3, **characterized in that** the additional visible light LED emits blue light.

5. The device according to any preceding claim, **characterized in that** the device is powered from an in-built battery or a charger of at least 1A.

6. The device according to any preceding claim, **characterized in that** the timer controlling unit (6) is adapted to set LED light source (2) illumination period to 300 s or 600 s with the accuracy of 0.5 s.

## Patentansprüche

1. Kompaktes UVB-Lichttherapiegerät für die Behandlung von Vitiligo, atopischer Dermatitis und Psoriasis, **dadurch gekennzeichnet, dass** es aus einem Gehäuse besteht, dass ein Netzteil enthält, das sich aus einem seriell angeschlossenen Netzschalter (5), einer Timer-Steuereinheit (6) und wenigstens einer LED-Leuchte (2) zusammensetzt, wobei die LED-Leuchte (2) über ein Kabel (4) mit einem Netzschalter (5) verbunden ist, wobei wenigstens eine LED-Leuchte (2) **dadurch gekennzeichnet, dass** sie Strahlung mit einer Wellenlänge von 310 nm in voller spektraler Breite bei einer Halbwertsbreite von 10 nm emittiert und eine optische Leistungsstärke im Bereich von 0,6 mW bis 1 mW hat, wobei gilt,
dass das Gerät einen abnehmbaren Abstandshalter (3) hat, der wenigstens eine LED-Leuchte (2) verdeckt,
dass der Abstandshalter in der Nähe von wenigstens einer LED-Leuchte am Gehäuse angebracht ist und eine Höhe von 0,5-1 cm hat, gemessen von der Gehäuseoberfläche bis zur Außenfläche des Abstandhalters.

2. Gerät nach Anspruch 1, **dadurch gekennzeichnet, dass** die LED-Leuchte (2) eine optische Leistung von 0,8 mW hat.

3. Gerät nach Anspruch 1, **dadurch gekennzeichnet, dass** in der Nähe der LED-Leuchte (2) eine zusätzliche LED installiert ist, die **dadurch gekennzeichnet ist, dass** sie sichtbares Licht emittiert.

4. Gerät nach Anspruch 3, **dadurch gekennzeichnet, dass** die zusätzliche sichtbares Licht emittierende LED derart eingestellt ist, dass sie blaues Licht emittiert.

5. Gerät nach einem der vorhergehenden Ansprüche, **dadurch gekennzeichnet, dass** es eine Stromversorgung in Form einer integrierten Batterie oder eines Ladegeräts mit mindestens 1 A hat.

6. Gerät nach einem der vorhergehenden Ansprüche, **dadurch gekennzeichnet, dass** die Timer-Steuereinheit (6) **dadurch gekennzeichnet ist, dass** die LED-Leuchte (2) eine Leuchtzeit von 300 s oder 600 s hat, wobei die Einstellungsgenauigkeit 0,5 s beträgt.

## Revendications

1. Un dispositif de luminothérapie UVB compact, pour le traitement du vitiligo, de la dermatite atopique et du psoriasis, comportant un boîtier contenant une alimentation de courant avec un interrupteur de puissance (5) monté avec une unité de minuterie (6) dans lequel au moins une source lumineuse LED (2) est reliée à l'interrupteur de puissance (5) par un fil (4),
dans lequel au moins une source LED (2) est adaptée pour émettre un rayonnement à une longueur d'onde de 310 nm avec une largeur spectrale complète à la moitié du maximum jusqu'à 10 nm et a une puissance optique comprise entre 0,6 mW et 1 mW,
et dans lequel le dispositif comporte une entretoise amovible (3) recouvrant au moins une source lumineuse LED (2),
fixé sur le boîtier à proximité d'au moins une source lumineuse LED et qui a une hauteur de 0,5 à 1 cm, mesurée de la surface du boîtier à la surface externe de l'entretoise.

2. Le dispositif selon la revendication 1, **caractérisé en ce que** la source lumineuse LED (2) a une puissance optique de 0,8 mW.

3. Le dispositif selon la revendication 1, **caractérisé en ce qu'**à proximité de la source lumineuse LED (2) est installée une LED lumineuse visible supplémentaire.

4. Le dispositif selon la revendication 3, **caractérisé en ce que** la LED lumineuse visible supplémentaire émet la lumière bleue.

5. Le dispositif selon toute revendication précédente, **caractérisé en ce que** le dispositif est alimenté par une batterie incorporée ou un chargeur d'au moins 1A.

6. Le dispositif selon toute revendication précédente, **caractérisé en ce que** l'unité de commande de minuterie (6) est adaptée pour régler la période d'éclairage de la source lumineuse LED (2) à 300 s ou 600 s avec une précision de 0,5 s.
